# EUROPEAN PATENT SPECIFICATION

(11) **EP 2 853 523 B1**
(45) Date of publication and mention of the grant of the patent: **30.05.2018**
(21) Application number: 14185927.2
(22) Date of filing: 23.09.2014
(51) Int. Cl.: C07C 41/30, C07C 17/263, C07C 25/18, C07C 43/29, C09K 19/12, C09K 19/30

(54) **Process for producing fluorine-containing biaryl compound**
Verfahren zur Herstellung einer fluorhaltigen Biarylverbindung
Procédé de production de composé biaryle contenant du fluor

(30) Priority: 30.09.2013 JP 2013204525
(43) Date of publication of application: 01.04.2015
(73) Proprietor: Daikin Industries, Ltd., Osaka 530-8323 (JP)
(72) Inventor: Nose, Masatoshi, Osaka, 530-8323 (JP); Ishihara, Sumi, Osaka, 530-8323 (JP); Nomura, Takashi, Osaka, 530-8323 (JP); Kishikawa, Yousuke, Osaka, 530-8323 (JP)
(74) Representative: Hoffmann Eitle

(56) References cited:
- EP-A1- 1 900 792
- BRUCE H. LIPSHUTZ ET AL: "Room-Temperature Suzuki-Miyaura Couplings in Water Facilitated by Nonionic Amphiphiles +", ORGANIC LETTERS, vol. 10, no. 7, 1 April 2008 (2008-04-01), pages 1333-1336, XP55164171, ISSN: 1523-7060, DOI: 10.1021/ol702714y
- BARDER TIMOTHY E ET AL: "Catalysts for Suzuki-Miyaura Coupling Processes: Scope and Studies of the Effect of Ligand Structure", JOURNAL OF THE AMERICAN CHEMICAL SOCIETY, AMERICAN CHEMICAL SOCIETY, vol. 127, no. 13, 1 January 2005 (2005-01-01), pages 4685-4696, XP002413444, ISSN: 0002-7863, DOI: 10.1021/JA042491J
- C. ZHANG ET AL.: "Palladium-Imidazol-2-yliden Complexes as catalysts for facile and efficient Suzuki Cross-Coupling reactions of aryl chlorides with arylboronic acids", JOURNAL OF ORGANIC CHEMISTRY, vol. 64, 5 June 1999 (1999-06-05), pages 3804-3805, XP002734969,
- NAJERA C ET AL: "Highly active oxime-derived palladacycle complexes for Suzuki-Miyaura and Ullmann-type coupling reactions", THE JOURNAL OF ORGANIC CHEMISTRY, AMERICAN CHEMICAL SOCIETY, US, vol. 67, 1 January 2002 (2002-01-01), pages 5588-5594, XP002285485, ISSN: 0022-3263, DOI: 10.1021/JO025619T

## Description

### Technical Field

The present invention relates to a process for producing a biaryl compound comprising a fluorine atom(s) as a substituent.

### Background Art

A biaryl compound comprising a fluorine atom(s) as a substituent is useful as a material for medicines and liquid crystals, and as an intermediate thereof.

One example of a process for producing a fluorine-containing biaryl compound is a process in which an aromatic boronic acid compound comprising a fluorine atom(s) as a substituent is subjected to a coupling reaction with an aromatic compound comprising, for example, a bromine atom, an iodine atom or an -OSO₂CF₃ group (see JP-A-2011-225566, JP-A-2011-195587, JP-A-2008-69153 and CN-A-102675062).

In this process, when an aromatic boronic acid compound comprising a fluorine atom at the ortho-position is used as a starting material, the starting material is unstable, and easily decomposes due to heating or influences attributable to water, oxygen, etc. Furthermore, an aromatic compound comprising, for example, a bromine atom, an iodine atom or an -OSO₂CF₃ group, that is used as the other starting material, is expensive, increasing the production cost.

In contrast, an aromatic chloride comprising a chlorine atom as a substituent is advantageous in that it is less expensive compared to an aromatic compound comprising, for example, a bromine atom, an iodine atom or an -OSO₂CF₃ group, and has a smaller molecular weight. If such an aromatic chloride can be efficiently reacted with the aromatic boronic acid compound comprising a fluorine atom at the ortho-position, this method will be industrially applicable as a process for producing a biaryl compound comprising a fluorine atom at the ortho-position.

However, an aromatic chloride comprising a chlorine atom as a substituent is less active and has poor reactivity compared to an aromatic compound comprising, for example, a bromine atom or an iodine atom. This makes it difficult to allow such a compound to efficiently react with a fluorine-containing aromatic boronic acid compound. Therefore, various methods for reacting them have been studied.

For example, Synthesis; 4, 692-698 (2006) below discloses that the target object can be obtained at a yield of 98% by reacting them in an H₂O solvent at 100°C for 2 hours using sodium hydroxide as a base and palladium carbon as a catalyst. However, in this method, the aromatic chloride used as a starting material is limited to highly reactive compounds that further comprise a nitro group as a substituent. Furthermore, because a large amount of tetrabutylammonium bromide (TBAB) is added, the production cost of such compounds is unduly high.

Organic Letters; 10(7), 1333-1336 (2008) below discloses that the target object can be obtained at a high yield by adding a specific nonionic surfactant and allowing the mixture to react in an H₂O solvent at 23°C for 22 hours in the presence of triethylamine (NEt₃) while using Pd(dtbpf)Cl₂, which is a Pd complex, as a catalyst. However, such a process requires the addition of expensive nonionic amphiphile to obtain a sufficient reaction yield. Furthermore, the aromatic boronic acid is used in an excessive (2 equivalent) amount relative to the aromatic chloride, and the amount of the catalyst used is as large as 2 mol%. This is also a costly method.

Furthermore, JACS127(13), 4685-4696 (2005); JACS, 132(40), 14073-14075 (2010); Angewandte Chemie, Int Ed; 44(38), 6173-6177 (2005) and Tetrahedron Lett.; 52(39), 5055-5059 (2011), as well as WO 2010/045258, WO 2007/031828 and other documents also disclose production examples utilizing a coupling reaction of an aromatic chloride with an aromatic boronic acid compound comprising a fluorine atom at the ortho-position. However, in order to achieve a high reaction yield, the aromatic boronic acid compound was used in an excessive amount relative to the aromatic chloride, and an unduly large amount of Pd catalyst was also used. Therefore, these production methods are not advantageous in terms of industrial applicability.

Angewandte Chemie, Int Ed; 44, 928-931 (2008) listed below discloses a method for obtaining a target object from a boronic acid, which is easily undergoes hydrolysis, by a coupling reaction. In this method, boronic acid is tetra-coordinated beforehand, and a reaction is performed in a nonaqueous solvent without using a base. However, this method requires using boronic acid, which has been made tetra-coordinated state beforehand; therefore, the handling of the boronic acid itself is problematic, and the production process becomes unduly long and complicated. Furthermore, because the addition of copper salt is necessary, this method is not advantageous in view of industrial applicability.

As described above, in the aforementioned methods, because the aromatic boronic acid compound is easily decomposed due to its instability, and the aromatic chloride is inactive, the aromatic boronic acid compound is used in an excessive amount relative to the aromatic chloride or a large amount of catalyst is used in order to achieve a high reaction yield. Furthermore, in these methods, various measures, such as heating, the use of expensive additives, tetra-coordinating the boronic acid beforehand, etc., are tried. This increases the production cost; therefor, these methods are not industrially advantageous.

### Summary of Invention

### Technical Problem

The present invention has been accomplished in view of the above-described problems of the prior art. A main object of the present invention is to provide a process that allows production of a biaryl compound comprising a fluorine atom at the ortho-position, using an inexpensive starting material, under economically advantageous conditions, and in an efficient manner.

### Solution to Problem

The present inventors conducted extensive research to achieve the above object. As a result, the inventors found that according to a process wherein an aromatic boronic acid compound comprising a fluorine atom at the ortho-position or a cyclic trimer thereof, and an aromatic chloride having a specific substituent are used as starting materials, and are reacted under heating in a water-containing solvent in the presence of a tertiary amine compound, using a palladium compound as a catalyst, the target fluorine-containing biaryl compound comprising a fluorine atom at the ortho-position can be produced at a high yield using only relatively inexpensive starting materials without using expensive additives. The present invention has been accomplished on the basis of this finding, and by conducting further research.

More specifically, the present invention provides a process for producing a fluorine-containing biaryl compound of Formula (III) : 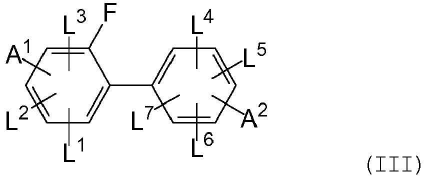 wherein
- L¹-L⁷: each independently are H, F or linear or branched C₁₋₆-alkyl;
- A₁: is H, F, linear or branched C₁₋₆-alkyl, a cyclic aliphatic hydrocarbon group optionally having one or more substituents, and further optionally having one or more hetero atoms, optionally substituted phenyl, or an optionally substituted aromatic heterocycle, and
- A₂: is H, F, -CN, -CF₃, -OCF₃, -O-A³, -COOA³, -CF₂OA³, or 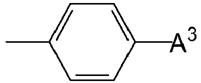
wherein A³ is H, linear or branched C₁₋₆-alkyl, phenyl, difluorophenyl, trifluorophenyl, difluoro trifluoromethyl phenyl or difluoro trifluoromethoxy phenyl.
the process comprises reacting at least one aromatic boronic acid compound of formula (I) or a cyclic trimer (Ia) formed by dehydration-condensation thereof: 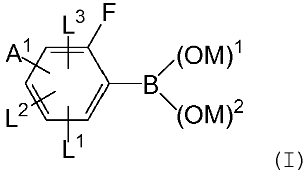 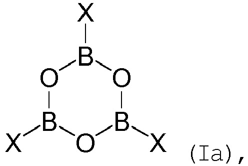 wherein X is 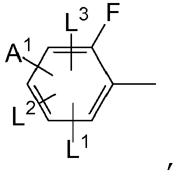 and wherein L¹-L³ and A¹ are the same as defined above, and M¹ and M² each independently are H, linear or branched C₁₋₆-alkyl, or a monovalent metal atom; or M¹ and M² bond to each other to form a bivalent aliphatic hydrocarbon group,
with an aromatic chloride of formula (II): 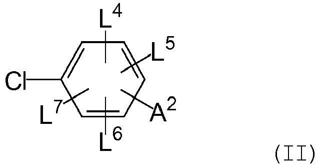 wherein L⁴-L⁷ and A² are the same as defined above,
in a water-containing solvent selected from water and mixtures of water and an organic solvent, at a reaction temperature of 60°C or more and in the presence of a palladium catalyst and a tertiary amine compound.

Preferred embodiments of the present process are as defined in the appended dependent claims and/or the following detailed description.

Hereunder, the process for producing a fluorine-containing biaryl compound of the present invention is explained.

### Starting material

Among the starting materials used in the present invention, a compound of Formula (I) below is used as the aromatic boronic acid compound:

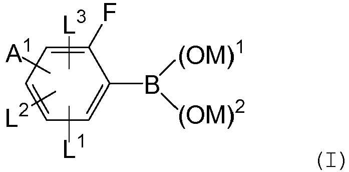

The aromatic boronic acid compound of Formula (I) is a compound comprising a fluorine atom at the ortho-position relative to the boronic acid group. The aromatic boronic acid compound is unstable in air, and easily decomposes due to heating or influences attributable to e.g. water or oxygen. However, by employing the conditions described below, the target fluorine-containing biaryl compound can be obtained at a high yield while preventing hydrolysis.

In the above Formula (I), L¹ to L³ are the same or different and each are H, F or lower alkyl. These groups are inactive to the reaction with the aromatic chloride of Formula (II) described below, and have little influence on the reaction.

A¹ is H, F, lower alkyl, a cyclic aliphatic hydrocarbon group optionally having one or more substituents, and further optionally having one or more hetero atoms, optionally substituted phenyl, or an optionally substituted aromatic heterocyclic group. In the boronic acid group, M¹ and M² are the same or different and each are H, lower alkyl or a monovalent metal atom. Furthermore, M¹ and M² may bond to each other to form a bivalent aliphatic hydrocarbon group, and may form a cyclic structure together with a boron atom. Examples of the bivalent aliphatic hydrocarbon group include linear or branched aliphatic C₂₋₆-hydrocarbon groups.

As the aromatic boronic acid compound of Formula (I), a compound in which A¹ is substituted at the para position relative to the boronic acid group is particularly preferable.

Among the groups represented by L¹ to L³ above, the lower alkyl includes linear or branched C₁₋₆-alkyl groups such as methyl, ethyl, n-propyl, isopropyl, n-butyl, isobutyl, tert-butyl, sec-butyl, n-pentyl, isopentyl, neopentyl, n-hexyl, isohexyl, and 3-methylpentyl group.

Examples of the lower alkyl represented by A¹ include those exemplified above as the lower alkyl represented by L¹ to L³ above. Among the groups represented by A¹, specific examples of the cyclic aliphatic hydrocarbon group optionally having one or more substituents, and further optionally having one or more hetero atoms include 4- to 6-membered cyclic aliphatic hydrocarbon groups such as cyclohexyl group, cyclopentyl group, cyclobutyl group and like C₄₋₆ cycloalkyl groups; heterocyclic hydrocarbon groups in which one, two, or more carbon atoms in these cycloalkyl groups are substituted with one, two, or more hetero atoms such as oxygen atom. Among the groups represented by A¹, the substituted or unsubstituted aromatic heterocyclic groups are not particularly limited, and examples thereof include 5- or 6-membered monocyclic aromatic heterocyclic groups. The heteroatoms in the aromatic heterocyclic groups are not particularly limited, and preferably at least one nitrogen atom is contained in the aromatic ring. Examples of such aromatic heterocyclic groups include pyrrolyl, pyridyl, imidazolyl, pyrazolyl, pyrazinyl, pyrimidinyl, pyridazinyl, isothiazolyl, and isoxazolyl.

Examples of the group that can function as a substituent for the cyclic aliphatic hydrocarbon group, phenyl, and aromatic heterocyclic group include F and lower alkyl. In this case, examples of the lower alkyl groups serving as substituents for the cyclic aliphatic hydrocarbon group, phenyl, and aromatic heterocyclic group include the same as those of the lower alkyl represented by L¹ to L³ described above. These substituents may substitute one, two, or more atoms or groups at arbitrary sites in each of the cyclic aliphatic hydrocarbon group, phenyl, and aromatic heterocyclic group.

In the boronic acid group, among the atoms or groups represented by M¹ and M², examples of the lower alkyl include the same as those of the lower alkyl represented by L¹ to L³. Examples of monovalent metal atom include alkali metals such as Na, K, and Li.

When M¹ and M² bond to each other to form a bivalent aliphatic hydrocarbon group, examples of the boronic acid group include the groups of the formulae below:

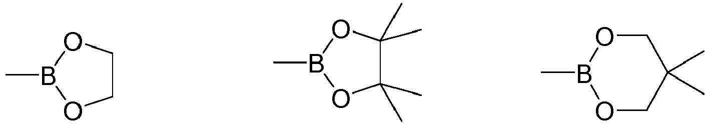

Each of the groups described above can be easily obtained by reacting, for example, boronic acid with ethylene glycol, or pinacol.

The aromatic boronic acid compound of Formula (I) takes the form of boronic acid, boronic ester, or boronic acid salt by comprising a boronic acid group -B(OM¹)(OM²).

The aromatic boronic acid compound of Formula (I) can also be used as a cyclic trimer obtained by subjecting the aromatic boronic acid compound to a dehydration-condensation reaction. Such cyclic trimers are referred to as a boroxine compound, represented by the chemical formula below. 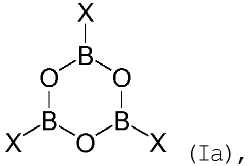 wherein X is 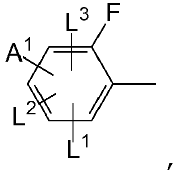 and wherein L¹-L³ and A¹ are the same as defined above

In the present invention, at least one boronic acid compound selected from the aromatic boronic acid compounds of Formula (I) described above and cyclic trimers formed by dehydration-condensation of the aromatic boronic acid compound may be used.

The aromatic boronic acid compound of Formula (I) and a cyclic trimer thereof are known compounds that are readily obtainable.

Among the starting materials, a compound of Formula (II) below is used as the aromatic chloride:

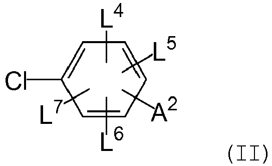

The aromatic chloride of Formula (II) is a compound having a relatively low reactivity with an aromatic boronic acid compound. However, by employing the reaction conditions described below, the coupling reaction with an aromatic boronic acid compound can proceed smoothly.

In Formula (II) above, L⁴ to L⁷ are the same or different and each represents H, F or lower alkyl. Among those groups, examples of the lower alkyl are the same as those of the lower alkyl represented by L¹ to L³ in Formula (I). A² is H, F,-CN, -CF₃, -OCF₃, -O-A³, -COOA³, -CF₂OA³, or a group of the formula below:

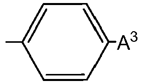

In each group above, the group A³ is H, lower alkyl, phenyl, or a group represented by the formula below:

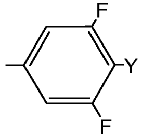

In the group shown above, Y is H, F, -CF₃, or -OCF₃.

The aromatic chloride of Formula (II) is a known compound that is readily obtainable.

As the aromatic chloride of Formula (II), a compound in which A² is substituted at the para position relative to Cl is particularly preferable.

### Process for producing fluorine-containing biaryl compound

The present invention requires reacting an aromatic chloride represented by Formula (II) with at least one boronic acid compound selected from aromatic boronic acid compounds of Formula (I) and cyclic trimers formed by dehydration-condensation of the aromatic boronic acid compound, in a water-containing solvent using a palladium compound as a catalyst in the presence of a tertiary amine compound.

This method uses as a starting material the boronic acid compound, which is unstable and easily undergoes hydrolysis, and the reaction is conducted in a water-containing solvent. However, by combining the conditions described above, the target fluorine-containing biaryl compound of Formula (III) can be obtained at a high yield while preventing hydrolysis of the starting material: 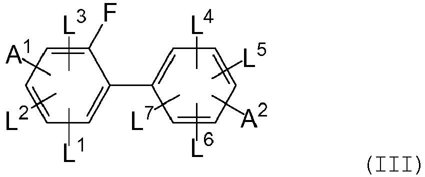 wherein L¹-L⁷, A¹, and A² are the same as defined above. Furthermore, the aromatic chloride of Formula (II) used as the starting material is relatively inexpensive, and the reaction proceeds without adding expensive additives and without adding a large amount of catalyst; accordingly, this method allows the target fluorine-containing biaryl compound to be obtained at a low cost.

In terms of the proportion of at least one boronic acid compound selected from the group consisting of the aromatic boronic acid compounds of Formula (I) and cyclic trimers formed by dehydration-condensation thereof to the aromatic chloride of Formula (II), the boronic acid compound is generally used in an excessive amount relative to the aromatic chloride in conventional methods because the boronic acid compound easily undergoes hydrolysis. However, in the process of the present invention, because the hydrolysis of the boronic acid compound can be suppressed, the target object can be obtained at a high yield even when the proportion of the boronic acid compound is reduced.

For example, per 1 mol of the aromatic chloride of Formula (II), the aromatic boronic acid compound of Formula (I) may be used in an amount of 0.8-1.5 mol, preferably 0.9-1.2 mol, more preferably 0.95-1.1 mol, and further more preferably 1.0-1.05 mol. When a boroxine compound, which is a cyclic trimer of the aromatic boronic acid compound, is used as the starting material, 1 mol of the cyclic trimer is regarded as 3 mol of the aromatic boronic acid compound represented by Formula (I).

As the water-containing solvent, water is used singly, or a mixture of water and an organic solvent is used. In the present invention, by conducting the reaction in the presence of the tertiary amine compound described below and in a water-containing solvent, the coupling reaction of the aromatic boronic acid compound with an aromatic chloride can proceed smoothly while suppressing the hydrolysis of the aromatic boronic acid compound.

The amount of water-containing solvent per 1 g of the aromatic chloride of Formula (II) may be, as the amount of water, ≥ 0.5 ml, for example, 0.5-20 ml, preferably 1-12 ml, and more preferably 2-8 ml.

As the organic solvents used in the mixed solvent, either a water-miscible organic solvent or an organic solvent which causes phase separation from water may be used. Specific examples thereof include DMAc (dimethylacetamide), DMF (dimethylformamide), acetonitrile, toluene, xylene, C₁₋₈ alcohols (e.g., ethanol and isopropanol), tetrahydrofuran (THF), and dioxane. The proportion of water to organic solvent in the mixed solvent is not particularly limited, and the content of water, relative to the total amount of water and the organic solvent, is preferably ≥ 20 vol.-%, more preferably 50-90 vol.-%, and further more preferably 60-80 vol.-%.

The present invention requires the use of a tertiary amine compound as a basic compound. By the use of a tertiary amine compound under the reaction conditions of the present invention, the target fluorine-containing biaryl compound can be obtained at a high yield while suppressing hydrolysis of the aromatic boronic acid compound of Formula (I) and a cyclic trimer thereof. In contrast, when a strong inorganic base is used, hydrolysis of the starting material easily proceeds, and the reaction yield is greatly lowered.

Examples of the tertiary amine compound include diethylmethylamine, trimethylamine, triethylamine (NEt₃), diisopropylethylamine, and triisopropylamine. Among these, triethylamine is particularly preferable.

The amount of the tertiary amine compound, relative to 1 mol of the aromatic boronic acid compound of Formula (I), is preferably 1-4 mol, and more preferably 1.1-2.5 mol. When the boroxine compound, which is a cyclic trimer of the aromatic boronic acid compound, is used as the starting material, 1 mol of the cyclic trimer is regarded as 3 mol of the aromatic boronic acid compound represented by Formula (I).

The present invention uses a palladium catalyst. As the palladium catalyst, known palladium catalysts that are effective in the coupling reaction between an aromatic boronic acid compound and aromatic halide can be used. In particular, the palladium catalyst used in the present invention is preferably a palladium complex which is formed in the reaction system by the presence of a zerovalent or divalent palladium and a ligand for palladium in the reaction system. By using such a palladium complex, the coupling reaction of the aromatic boronic acid compound of Formula (I) with the aromatic chloride of Formula (II) proceeds smoothly. The compound that serves as the source of the zerovalent or divalent palladium and the ligand may be separately added to the water-containing solvent, which is the reaction solvent, so as to form a palladium complex in the reaction system. Alternatively, a palladium complex, which has been formed from palladium and a ligand, may be added to the water-containing solvent.

Examples of the source of the zerovalent or divalent palladium include palladium carbon, in which Pd is supported on activated carbon; metal palladium-supported material, in which palladium is supported on a carrier such as silica, alumina or zeolite; and divalent palladium-containing palladium salts, such as palladium chloride, palladium acetate, palladium bromide, and palladium iodide.

As the ligand for palladium, known ligands capable of forming a palladium complex usable in the coupling reaction can be used. In particular, the use of an electron-rich tertiary phosphine-based ligand that comprises at least one alkyl group, and further comprises a bulky substituent is preferable.

Specific examples of such tertiary phosphine-based ligands include the following compounds:

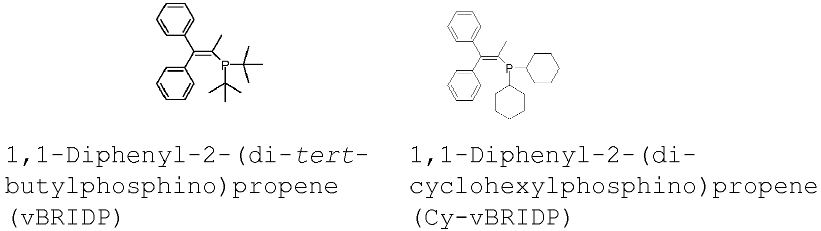

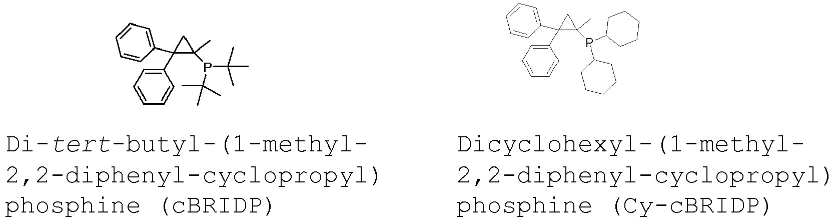

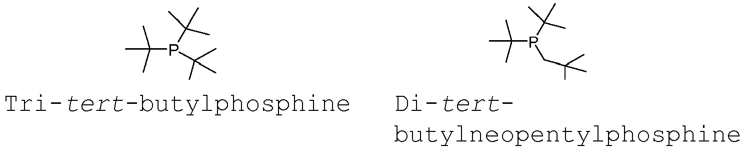

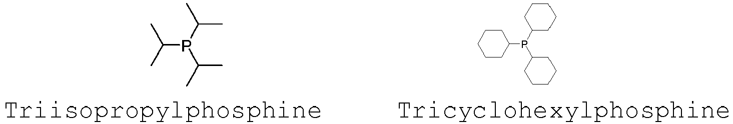

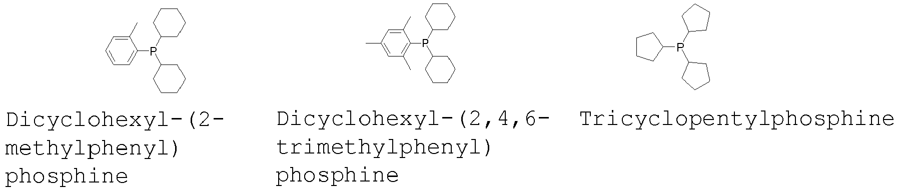

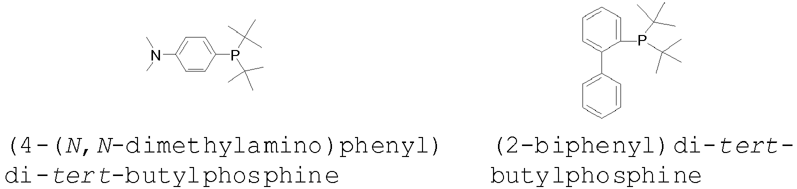

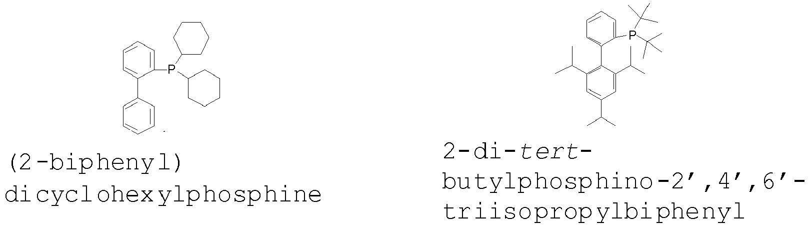

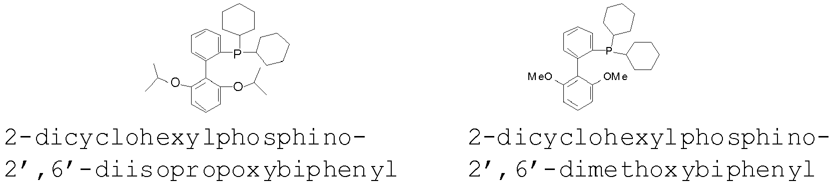

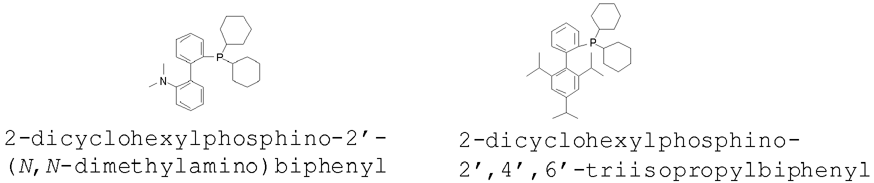

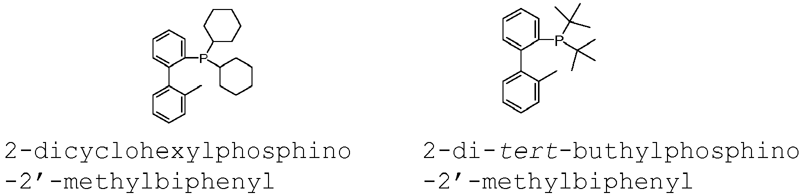

When the compound that serves as the source of palladium and a ligand are separately added to the reaction mixture, the amount of the ligand is not particularly limited, and is preferably 1-4 mol per 1 mol of palladium.

In the present invention, by employing the above conditions, the coupling reaction can proceed smoothly with an amount of palladium catalyst smaller than that used in conventional methods. For example, the amount of the palladium catalyst, calculated as the amount of palladium atoms contained in the palladium catalyst, is as small as ≥ 0.00005 mol relative to 1 mol of the aromatic chloride of Formula (II); the use of such an extremely small amount of palladium catalyst can allow the reaction to proceed smoothly. There is no particular upper limitation on the amount of the palladium catalyst used, as the use of a large amount of palladium catalyst does not adversely affect the progress of the reaction. However, the use of an unduly large amount of catalyst increases the cost; therefore, the amount of the palladium catalyst, calculated as the amount of palladium atoms, may be generally ≤ 0.004 mol relative to 1 mol of the aromatic chloride.

In the present invention, a phase transfer catalyst may be used, if necessary. By the use of a phase transfer catalyst, the reaction yield can be improved.

The phase transfer catalyst is not particularly limited. Examples thereof include quaternary ammonium salts, such as benzyltriethylammonium bromide, benzyltrimethylammonium chloride, hexadecyltriethylammonium bromide, hexadecyltriethylammonium bromide, hexadecyltrimethylammonium chloride, dodecyltriethylammonium chloride, octyltriethylammonium bromide, tetra-n-butylammonium bromide, tetra-n-butylammonium chloride, tetraethylammonium chloride, and trioctylmethylammonium chloride; quaternary phosphonium salts, such as hexadecyltriethylphosphonium bromide, hexadecyltributylphosphonium chloride, tetra-n-butylphosphonium bromide, tetra-n-butylphosphonium chloride, trioctylethylphosphonium bromide, and tetraphenylphosphonium bromide; and crown ethers, such as 18-crown-6, dibenzo-18-crown-6, and dicyclohexyl-18-crown-6.

The amount of the phase transfer catalyst used is not particularly limited and may be selected from the range of preferably 0.005-0.3 mol, and more preferably 0.01-0.1 mol per 1 mol of the aromatic chloride represented by Formula (II).

In the present invention, it is necessary that the coupling reaction of the aromatic chloride of Formula (II) with at least one boronic acid compound selected from the group consisting of aromatic boronic acid compounds of Formula (I) and cyclic trimers formed by dehydration-condensation of the aromatic boronic acid compound be conducted under heating. By conducting the reaction under heating in the presence of a tertiary amine compound, the reaction yield can be improved without using expensive additives, while suppressing the decomposition of the aromatic boronic acid compound that is used as a starting material. Specifically, the reaction temperature is ≥ 60°C, preferably 75-125°C, and more preferably 85-110°C.

There is no particular limitation on the pressure applied during the reaction, which can be conducted under a wide range of pressure from reduced pressure to increased pressure. Usually, the reaction may be conducted under an ordinary pressure to an increased pressure such as 0.1-0.4 MPa. When a glass reactor is used as the reaction vessel, the pressure is preferably in the range from reduced pressure to ordinary pressure; and when an autoclave is used, the reaction is preferably conducted under increased pressure.

The method described above allows the fluorine-containing biaryl compound of Formula (III) below to be obtained at a high yield: 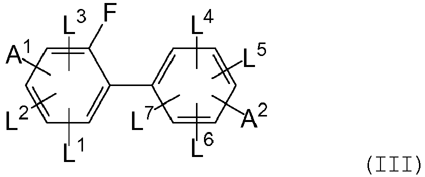 wherein L¹-L⁷, A¹, and A² are the same as defined above.

The fluorine-containing biaryl compound obtained by the above process can be purified and collected by known methods such as column chromatography or recrystallization.

### Advantageous Effects of Invention

The present invention makes it possible to obtain the target fluorine-containing biaryl compound at a high yield, using a relatively inexpensive starting material without using expensive additives, and further without using a large amount of aromatic boronic acid compound or catalyst.

Therefore, the present invention is industrially advantageous as a process for producing a biaryl compound comprising a fluorine atom at the ortho-position.

### Description of Embodiments

Hereunder, the present invention is explained in detail with reference to Examples.

### Example 1

### Synthesis of 3,5,2'-trifluoro-4"-propyl-[1,1';4',1"]terphenyl

4.31 g (29.0 mmol) of 1-chloro-3,5-difluorobenzene and 7.64 g (29.6 mmol, 1.02 equivalent amount) of 4'-propyl-3-fluoro-4-biphenylboronic acid were placed in a 200-ml SUS autoclave, after which 468 mg (0.05 equivalent amount) of tetra-n-butyl ammonium bromide (hereunder referred to as "TBAB"), 5.5 mg (0.0004 equivalent amount) of 2-dicyclohexylphosphino-2',4',6'-triisopropylbiphenyl (purchased from Johnson Matthey Japan Inc.), and 24.7 mg (0.0002 equivalent amount) of 5% palladium carbon (50% moisture content, manufactured by N.E. Chemcat Corporation; E-Type) were placed in the autoclave. The autoclave was subsequently sealed, and the atmosphere therein was replaced with nitrogen. Thereafter, 30 ml of deaerated water and 10 ml of deaerated toluene were added thereto, and 4.4 g (1.5 equivalent amount) of triethylamine was further added thereto, followed by stirring at the reaction liquid temperature of 95°C for 3 hours.

The mixture was stirred at room temperature for 20 minutes, and 30 ml of toluene and 20 ml of water were added to the reaction mixture, followed by additional stirring for 15 minutes. The reaction yield at this point was 99.8% (quantified by ¹⁹F NMR). The organic layer was subjected to suction filtration (using Celite), the filtrate was concentrated, and then the residue was purified using column chromatography. The crude substance thus obtained was recrystallized with a mixed solvent of n-heptane and denatured ethanol, thereby obtaining 3,5,2'-trifluoro-4"-propyl-[1,1';4',1"]terphenyl in the form of a white solid.
Amount obtained: 8.43 g (isolation yield 89%)
Melting point: 59°C
¹⁹F-NMR (376MHz, CDCl₃) δ -110.2 (t, J = 7.6Hz, 2F), -117. 6 (d, J = 12.0Hz, 1F).

### Example 2

### Synthesis of 2,3',5'-trifluoro-4-propylbiphenyl

8.62 g (58.0 mmol) of 1-chloro-3,5-difluorobenzene and 10.77 g (59.2 mmol, 1.02 equivalent amount) of 2-fluoro-4-propylphenylboronic acid were placed in a 200-ml SUS autoclave, after which 935 mg (0.05 equivalent amount) of TBAB and 20.5 mg (0.0005 equivalent amount) of bis(di-tert-butyl(4-dimethylaminophenyl)phosphine)dichloropalladium (II) (purchased from Johnson Matthey Japan Inc.) were placed in the autoclave. The autoclave was subsequently sealed, and the atmosphere therein was replaced with nitrogen. Thereafter, 50 ml of deaerated water was added thereto, and 8.8 g (1.5 equivalent amount) of triethylamine was further added thereto, followed by stirring at the reaction liquid temperature of 85°C for 2 hours.

The mixture was stirred at room temperature for 20 minutes, and 30 ml of toluene was added to the reaction mixture, followed by additional stirring for 15 minutes. The reaction yield at this point was 99.2% (quantified by ¹⁹F NMR) . The organic layer was subjected to suction filtration (using Celite), the filtrate was concentrated, and then the residue was purified using column chromatography. The oily substance obtained was subjected to distillation under reduced pressure, thereby obtaining 2,3',5'-trifluoro-4-propylbiphenyl in the form of a colorless liquid.
Amount obtained: 12.1 g (isolation yield 83%)
¹⁹F-NMR (376MHz, CDCl₃) δ -110.3(t, J = 7.6Hz, 2F), -117.0(d, J = 12.0Hz, 1F).

### Example 3

### Synthesis of 4-[difluoro-(3,4,5-trifluoro-phenoxy)-methyl]-2',3,5-trifluoro-4"-propyl-[1,1';4',1"]terphenyl

### Example 3-1

1.00 g (2.90 mmol) of 5-[4-chloro-2,6-difluorophenyl)difluoromethoxy]-1,2,3-trifluorobenzene and 0.764 g (2.96 mmol, 1.02 equivalent amount) of 4'-propyl-3-fluoro-4-biphenylboronic acid were placed in a 50-ml SUS autoclave, after which 46.7 mg (0.05 equivalent amount) of TBAB and 1.0 mg (0.0005 equivalent amount) of bis(di-tert-butyl(4-dimethylaminophenyl)phosphine)dichloropalladium (II) (purchased from Johnson Matthey Japan Inc.) were placed in the autoclave. The autoclave was subsequently sealed, and the atmosphere therein was replaced with nitrogen.

The 5-[4-chloro-2,6-difluorophenyl)difluoromethoxy]-1,2,3-trifluorobenzene used as the starting material is a compound that can be easily obtained by the methods described in WO 2001/064667 A1, JP1998-273294A, or elsewhere.

Thereafter, 4.0 ml of deaerated water was added thereto, and 0.44 g (1.5 equivalent amount) of triethylamine was further added thereto, followed by stirring at the reaction liquid temperature of 85°C for 2 hours. The mixture was stirred at room temperature for 20 minutes, and 4.0 ml of toluene and 2.0 ml of water were added to the reaction mixture, followed by additional stirring for 15 minutes. The reaction yield at this point was 98.8% (quantified by ¹⁹F NMR). The organic layer was subjected to suction filtration (using Celite), the filtrate was concentrated, and then the residue was purified using column chromatography. The crude substance thus obtained was recrystallized with a mixed solvent of n-heptane and denatured ethanol, thereby obtaining 4-[difluoro-(3,4,5-trifluoro-phenoxy)-methyl]-2',3,5-trifluoro-4"-propyl-[1,1';4',1"]terphenyl in the form of a white solid.
Amount obtained: 1.44 g (isolation yield 95.0%)
Melting point: 85°C
¹H-NMR(254MHz, CDCl₃) δ 7.72-7.46(m, 7H), 7.34-7.19(m, 4H), 2.67-2.61(t, J = 7.6Hz, 2H), 1.74-1.60(m, 2H), 0.98-0.92(t, J = 7.6Hz, 3H), ¹⁹F-NMR (254MHz, CDCl₃) δ -60.8 (t, J = 26.7Hz, 2F), -110.6 to -111.6(m, 2F), -117.0(dd, J = 12.7, 8.4Hz, 1F), -133.2 to - 133.4 (m, 2F), -164.1 to -164.3 (m, 1F) .

### Example 3-2

1.00 g (2.90 mmol) of 5-[4-chloro-2,6-difluorophenyl)difluoromethoxy]-1,2,3-trifluorobenzene and 0.765 g (2.96 mmol, 1.02 equivalent amount) of 4'-propyl-3-fluoro-4-biphenylboronic acid were placed in a 50-ml SUS autoclave, after which 46.9 mg (0.05 equivalent amount) of TBAB, 0.68 mg (0.0006 equivalent amount) of 1,1-diphenyl-2-(dicyclohexylphosphino)propene) (purchased from Takasago International Corporation), and 2.47 mg (0.0002 equivalent amount) of 5% palladium carbon (50% moisture content, manufactured by N.E. Chemcat Corporation; E-Type) were placed in the autoclave. The autoclave was subsequently sealed, and the atmosphere therein was replaced with nitrogen. Thereafter, 3.0 ml of deaerated water and 1.0 ml of deaerated N,N-dimethylacetamide (hereunder referred to as "DMAc") were added thereto, and 0.44 g (1.5 equivalent amount) of triethylamine was further added thereto, followed by stirring at the reaction liquid temperature of 94°C for 4 hours. The mixture was stirred at room temperature for 20 minutes, and 5.0 ml of toluene and 2.0 ml of water were added to the reaction mixture, followed by additional stirring for 15 minutes. The reaction yield was 96.6% (quantified by ¹⁹F NMR) .

### Example 3-3

200 g (580 mmol) of 5-[4-chloro-2,6-difluorophenyl)difluoromethoxy]-1,2,3-trifluorobenzene and 153 g (593 mmol, 1.02 equivalent amount) of 4'-propyl-3-fluoro-4-biphenylboronic acid were placed in a 3.0-L SUS autoclave, after which 9.34 g (0.05 equivalent amount) of TBAB, 111 mg (0.0004 equivalent amount) of 2-dicyclohexylphosphino-2',4',6'-triisopropylbiphenyl (purchased from Johnson Matthey Japan Inc.), and 494 mg (0.0002 equivalent amount) of 5% palladium carbon (50% moisture content, manufactured by N.E. Chemcat Corporation; E-Type) were placed in the autoclave. The autoclave was subsequently sealed, and the atmosphere therein was replaced with nitrogen. Thereafter, 600 ml of deaerated water and 200 ml of deaerated toluene were added thereto, and 88.2 g (1.5 equivalent amount) of triethylamine was further added thereto, followed by stirring at the reaction liquid temperature of 95°C for 3 hours.

The mixture was allowed to cool to room temperature and then stirred for 60 minutes. Thereafter, 400 ml of toluene and 200 ml of water were added to the reaction mixture, followed by additional stirring for 20 minutes. The reaction yield at this point was 99.7% (quantified by ¹⁹F NMR). The organic layer was subjected to suction filtration (using Celite), the filtrate was concentrated, and then the residue was purified using column chromatography. The crude substance thus obtained was recrystallized with denatured ethanol, thereby obtaining 4-[difluoro-(3,4,5-trifluoro-phenoxy)-methyl]-2',3,5-trifluoro-4"-propyl-[1,1';4',1"]terphenyl in the form of a white solid. Amount obtained: 292 g (isolation yield 96.4%, purity 99.9%).

### Example 3-4

1.00 g (2.90 mmol) of 5-[4-chloro-2,6-difluorophenyl)difluoromethoxy]-1,2,3-trifluorobenzene and 0.764 g (2.96 mmol, 1.02 equivalent amount) of 4'-propyl-3-fluoro-4-biphenylboronic acid were placed in a 50-ml SUS autoclave, after which 46.7 mg (0.05 equivalent amount) of TBAB, 1.10 mg (0.0008 equivalent amount) of 2-dicyclohexylphosphino-2',4',6'-triisopropylbiphenyl (purchased from Johnson Matthey Japan Inc.), and 4.94 mg (0.0004 equivalent amount) of 5% palladium carbon (50% moisture content, manufactured by N.E. Chemcat Corporation; E-Type) were placed in the autoclave. The autoclave was subsequently sealed, and the atmosphere therein was replaced with nitrogen. Thereafter, 3.0 ml of deaerated water and 1.0 ml of deaerated DMA_{C} were added thereto, and 0.44 g (1.5 equivalent amount) of triethylamine was further added thereto, followed by stirring at the reaction liquid temperature of 95°C for 3 hours. The mixture was stirred at room temperature for 20 minutes, and 5.0 ml of toluene and 2.0 ml of water were added to the reaction mixture, followed by additional stirring for 15 minutes. The reaction yield was 99.4% (quantified by ¹⁹F NMR) .

### Example 3-5

The reaction was conducted in the same manner as in Example 3-4 except that the reaction liquid was stirred at the reaction liquid temperature of 115°C, in place of 95°C, for 3 hours. The results revealed that the reaction yield was 97.0% (quantified by ¹⁹F NMR) .

### Example 3-6

The reaction was conducted in the same manner as in Example 3-4 except that the reaction liquid was stirred at the reaction liquid temperature of 105°C, in place of 95°C, for 3 hours. The results revealed that the reaction yield was 98.4% (quantified by ¹⁹F NMR) .

### Example 3-7

The reaction was conducted in the same manner as in Example 3-4 except that the reaction liquid was stirred at the reaction liquid temperature of 85°C, in place of 95°C, for 3 hours. The results revealed that the reaction yield was 91.9% (quantified by ¹⁹F NMR).

### Example 3-8

The reaction was conducted in the same manner as in Example 3-4 except that the reaction liquid was stirred at the reaction liquid temperature of 75°C, in place of 95°C, for 3 hours. The results revealed that the reaction yield was 78.4% (quantified by ¹⁹F NMR).

### Example 3-9

The reaction was conducted in the same manner as in Example 3-4 except that the reaction liquid was stirred at the reaction liquid temperature of 65°C, in place of 95°C, for 4 hours. The results revealed that the reaction yield was 59.1% (quantified by ¹⁹F NMR).

### Comparative Example 1

The reaction was conducted in the same manner as in Example 3-4 except that the reaction liquid was stirred at room temperature (20°C), in place of 95°C, for 3 hours. The results revealed that the target object was not detected at all (quantified by ¹⁹F NMR).

### Comparative Example 2

The reaction was conducted in the same manner as in Example 3-4 except that potassium hydroxide (2.4 equivalent amount) was used in place of triethylamine. The results revealed that the reaction yield was 32.2% (quantified by ¹⁹F NMR).

### Comparative Example 3

The reaction was conducted in the same manner as in Example 3-4 except that potassium phosphate (2.4 equivalent amount) was used in place of triethylamine. The results revealed that the reaction yield was 27.9% (quantified by ¹⁹F NMR).

### Comparative Example 4

The reaction was conducted in the same manner as in Example 3-4 except that 4.0 ml of DMAc alone was used in place of a mixed solvent of water and DMAc. The results revealed that the reaction yield was 6.6% (quantified by ¹⁹F NMR).

### Comparative Example 5

The reaction was conducted in the same manner as in Example 3-4 except that 4.0 ml of toluene alone was used in place of the mixed solvent of water and DMAc. The results revealed that the reaction yield was 33.9% (quantified by ¹⁹F NMR) .

### Example 4

### Synthesis of 4-[difluoro-(3,4,5-trifluoro-phenoxy)-methyl]-2',3,5-trifluoro-4"-pentyl-[1,1';4',1"]terphenyl

10.0 g (29.0 mmol) of 5-[4-chloro-2,6-difluorophenyl)difluoromethoxy]-1,2,3-trifluorobenzene and 8.47 g (29.6 mmol, 1.02 equivalent amount) of 4'-pentyl-3-fluoro-4-biphenylboronic acid were placed in a 300-ml SUS autoclave, after which 467 mg (0.05 equivalent amount) of TBAB, 16.6 mg (0.0012 equivalent amount) of 2-dicyclohexylphosphino-2',4',6'-triisopropylbiphenyl (purchased from Johnson Matthey Japan Inc.), and 49.4 mg (0.0004 equivalent amount) of 5% palladium carbon (50% moisture content, manufactured by N.E. Chemcat Corporation; E-Type) were placed in the autoclave. The autoclave was subsequently sealed, and the atmosphere therein was replaced with nitrogen. Thereafter, 30 ml of deaerated water and 10 ml of deaerated DMAc was added thereto, and 4.5 g (1.5 equivalent amount) of triethylamine was further added thereto, followed by stirring at the reaction liquid temperature of 99°C for 4 hours.

The mixture was stirred at room temperature for 20 minutes, and 50 ml of toluene and 30 ml of water were added to the reaction mixture, followed by additional stirring for 15 minutes. The reaction yield was 97.7% (quantified by ¹⁹F NMR). The organic layer was subjected to suction filtration (using Celite), the filtrate was concentrated, and then the residue was purified using column chromatography. The crude substance thus obtained was recrystallized with a mixed solvent of denatured ethanol and heptane, thereby obtaining 4-[difluoro-(3,4,5-trifluoro-phenoxy)-methyl]-2',3,5-trifluoro-4"-pentyl-[1,1';4',1"]terphenyl in the form of a white solid.
Amount obtained: 14.8 g (isolation yield 93.0%, purity 99.9%).
Melting point: 52°C.
¹H-NMR(254MHz, CDCl₃) δ 7.54-7.25 (m, 9H), 7.01-6.98(dd, J = 7.8, 5.8Hz, 2H), 2.68-2.64(t, J = 7.8Hz, 2H), 1.70-1.63(m, 2H), 1.38-1.32 (m, 4H), 0.93-0.90 (t, J = 6.8Hz, 3H), ¹⁹F-NMR (254MHz, CDCl₃) δ -62.0(t, J = 25.9Hz, 2F), -110.9 to -111.1(td, J = 26.7, 10.8Hz, 2F), -117.1(dd, J = 12.9, 12.7Hz, 1F), -132.8(m, 2F), -163.4 to -163.6(m, 1F) .

### Example 4-2

The reaction was conducted in the same manner as in Example 4 except that the reaction liquid was stirred at the reaction liquid temperature of 85°C, in place of 99°C, for 5 hours. The results revealed that the reaction yield was 74.2% (quantified by ¹⁹F NMR) .

### Example 5

### Synthesis of 4-[difluoro-(3,4,5-trifluoro-phenoxy)-methyl]-3,5,2'-trifluoro-4'-propylbiphenyl

1.00 g (2.90 mmol) of 5-[4-chloro-2,6-difluorophenyl)difluoromethoxy]-1,2,3-trifluorobenzene and 0.539 g (2.96 mmol, 1.02 equivalent amount) of 2-fluoro-4-propylphenylboronic acid were placed in a 50-ml SUS autoclave, after which 4.67 mg (0.05 equivalent amount) of TBAB, 5.5 mg (0.0004 equivalent amount) of 2-dicyclohexylphosphino-2',4',6'-triisopropylbiphenyl (purchased from Johnson Matthey Japan Inc.), and 2.47 mg (0.0002 equivalent amount) of 5% palladium carbon (50% moisture content, manufactured by N.E. Chemcat Corporation; E-Type) were placed in the autoclave. The autoclave was subsequently sealed, and the atmosphere therein was replaced with nitrogen. Thereafter, 3.0 ml of deaerated water and 1.0 ml of deaerated toluene were added thereto, and 0.44 g (1.5 equivalent amount) of triethylamine was further added thereto, followed by stirring at the reaction liquid temperature of 95°C for 3 hours.

The mixture was stirred at room temperature for 15 minutes, and 3.0 ml of toluene and 2.0 ml of water were added to the reaction mixture, followed by additional stirring for 20 minutes. The reaction yield at this point was 99.5% (quantified by ¹⁹F NMR). The organic layer was subjected to suction filtration (using Celite), the filtrate was concentrated, and then the residue was purified using column chromatography. The crude substance thus obtained was recrystallized with methanol, thereby obtaining 4-[difluoro-(3,4,5-trifluoro-phenoxy)-methyl]-3,5,2'-trifluoro-4'-propyl-biphenyl in the form of a white solid.
Amount obtained: 1.22 g (isolation yield 93.9%)
Melting point: 48°C
¹⁹F-NMR (376MHz, CDCl₃) δ -60.8 (t, J = 26.7Hz, 2F), -110.2 (t, J = 7.6Hz, 2F), -117.6(d, J = 12.0Hz, 1F), -133.2 to -133.4(m, 2F), -164.1 to -164.3(m, 1F).

### Example 6

### Synthesis of 3,5,2'-trifluoro-4"-propyl-[1,1';4',1"]terphenyl-4-carboxylic acid 3,4,5-trifluoro-phenyl ester

The reaction was conducted in the same manner as in Example 5 except that the starting materials were changed to the combination of 4-chloro-2,6-difluoro-benzoic acid 3,4,5-trifluoro-phenyl ester) with 4'-propyl-3-fluoro-4-biphenylboronic acid. The results revealed that the reaction yield was 98.6%.
¹⁹F-NMR (376MHz, CDCl₃) δ -108.7(d, J = 10.2Hz, 2F), -116.8(d, J = 10.2Hz, 1F), -132.6 to -132.7(m, 2F), -163.0(m, 1F).

### Example 7

### Synthesis of 4-[(3,5-difluorophenoxy)-difluoromethyl]-3,5,2'-trifluoro-4"-propyl-[1,1';4',1"]terphenyl

The reaction was conducted in the same manner as in Example 5 except that the starting materials were changed to the combination of 5-[4-chloro-2,6-difluorophenyl)difluoromethoxy]-1,3-difluorobenzene with 4'-propyl-3-fluoro-4-biphenylboronic acid. The results revealed that the reaction yield was 99.6%.
¹⁹F-NMR (376MHz, CDCl₃) δ -61.6 to -61.7 (t, J = 25.9Hz, 2F), -108.5 (t, J = 7.9Hz, 2F), -110.8 to -111.0 (m, 2F), -117.1 (d, J = 8.6Hz, 1F).

### Example 8

The reaction was conducted in the same manner as in Example 5 except that the starting materials were changed to the combination shown in the reaction scheme below. The results revealed that the reaction yield of the target object was 98.0%.

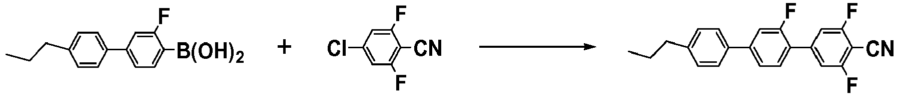

### Example 9

The reaction was conducted in the same manner as in Example 5 except that the starting materials were changed to the combination shown in the reaction scheme below. The results revealed that the reaction yield of the target object was 92.2%.

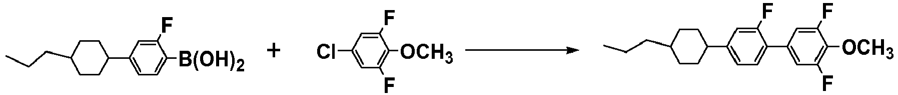

## Claims

1. A process for producing a fluorine-containing biaryl compound of Formula (III): 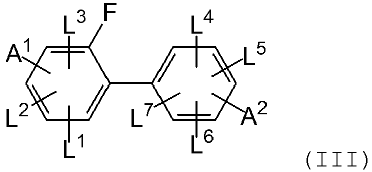 wherein
L¹-L⁷ each independently are H, F or linear or branched C₁₋₆-alkyl;
A¹ is H, F, linear or branched C₁₋₆-alkyl, a cyclic aliphatic hydrocarbon group optionally having one or more substituents, and further optionally having one or more hetero atoms, optionally substituted phenyl, or an optionally substituted aromatic heterocycle, and
A² is H, F, -CN, -CF₃, -OCF₃, -O-A³, -COOA³, -CF₂OA³, or 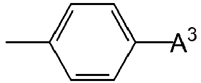 wherein A³ is H, linear or branched C₁₋₆-alkyl, phenyl, difluorophenyl, trifluorophenyl, difluoro trifluoromethyl phenyl or difluoro trifluoromethoxy phenyl.
the process comprises reacting at least one aromatic boronic acid compound of formula (I) or a cyclic trimer (Ia) formed by dehydration-condensation thereof: 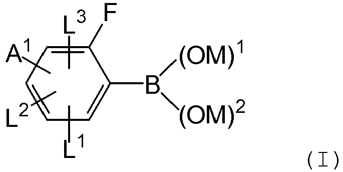 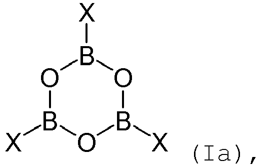 wherein X is 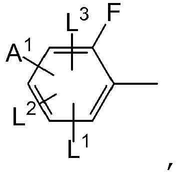 and wherein L¹-L³ and A¹ are the same as defined above, and M¹ and M² each independently are H, linear or branched C₁₋₆-alkyl, or a monovalent metal atom; or M¹ and M² bond to each other to form a bivalent aliphatic hydrocarbon group,
with an aromatic chloride of formula (II): 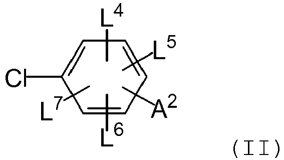 wherein L⁴-L⁷ and A² are the same as defined above,
in a water-containing solvent selected from water and mixtures of water and an organic solvent, at a reaction temperature of 60°C or more and in the presence of a palladium catalyst and a tertiary amine compound.

2. The process of claim 1, wherein compound (I) has the following formula, with L¹-L³ and A¹ being as defined in claim 1: 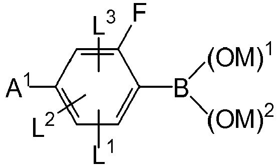 and compound (II) has the following formula, with L⁴-L⁷ and A² being as defined in claim 1:

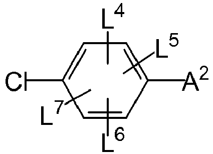

3. The process of any of claims 1-2, wherein the tertiary amine compound is triethylamine.

4. The process of any of claims 1-3, wherein the palladium catalyst is a palladium complex formed of a zerovalent or divalent palladium and a tertiary phosphine-based ligand.

5. The process of claim 4, wherein the palladium complex is one which is formed in a water-containing solvent by adding a tertiary phosphine-based ligand and a compound that serves as a source for a zerovalent or divalent palladium to the water-containing solvent, or the palladium complex is one which has been formed beforehand from a zerovalent or divalent palladium and tertiary phosphine-based ligand.

6. The process of any of claims 1-5, wherein the reaction is performed in the presence of a phase transfer catalyst.

## Patentansprüche

1. Verfahren zur Herstellung einer fluorhaltigen Biarylverbindung der Formel (III): 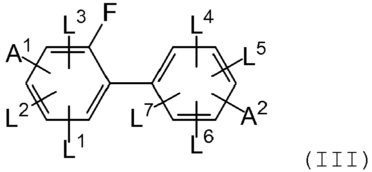 worin
L¹ bis L⁷ jeweils unabhängig H, F oder geradkettiges oder verzweigtes C₁₋₆-Alkyl sind;
A¹ H, F, geradkettiges oder verzweigtes C₁₋₆-Alkyl, eine cyclische aliphatische Kohlenwasserstoffgruppe, die gegebenenfalls einen oder mehrere Substituenten aufweist und ferner gegebenenfalls ein oder mehrere Heteroatom(e) aufweist, gegebenenfalls substituiertes Phenyl oder ein gegebenenfalls substituierter aromatischer Heterocyclus ist und
A² H, F, -CN, -CF₃, -OCF₃, -O-A³, -COOA³, -CF₂OA³, oder 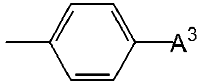 ist, worin A³ H, geradkettiges oder verzweigtes C₁₋₆-Alkyl, Phenyl, Difluorphenyl, Trifluorphenyl, Difluortrifluormethylphenyl oder Difluortrifluormethoxyphenyl ist;
wobei das Verfahren die Umsetzung zumindest einer aromatischen Boronsäureverbindung der Formel (I) oder eines cyclischen Trimers (Ia), das durch Dehydrierungskondensation hiervon gebildet ist: 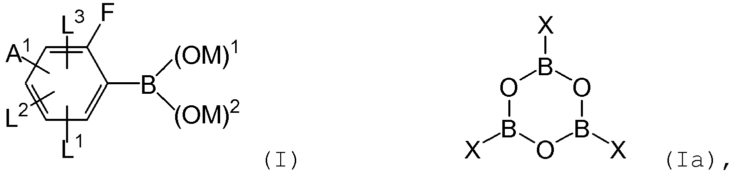 worin X 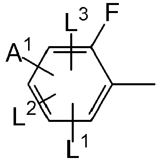 ist,
und worin L¹ bis L³ und A¹ die gleichen sind, wie oben definiert, und M¹ und M² jeweils unabhängig H, geradkettiges oder verzweigtes C₁₋₆-Alkyl oder ein monovalentes Metallatom sind; oder M¹ und M² miteinander verbunden sind, um eine bivalente aliphatische Kohlenwasserstoffgruppe zu bilden,
mit einem aromatischen Chlorid der Formel (II): 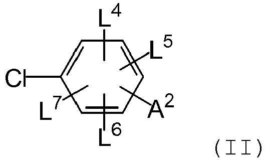 worin L⁴ bis L⁷ und A² die gleichen sind, wie oben definiert,
in einem wasserhaltigen Lösungsmittel, ausgewählt aus Wasser und Mischungen von Wasser und einem organischen Lösungsmittel, bei einer Reaktionstemperatur von 60°C oder mehr und in Gegenwart eines Palladiumkatalysators und einer tertiären Aminverbindung umfasst.

2. Verfahren gemäss Anspruch 1, wobei die Verbindung (I) die nachstehende Formel aufweist, worin L¹ bis L³ und A¹ wie in Anspruch 1 definiert sind: 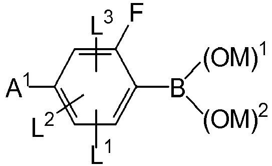 und die Verbindung (II) die nachstehende Formel aufweist, worin L⁴ bis L⁷ und A² wie in Anspruch 1 definiert sind:

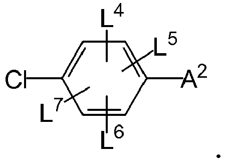

3. Verfahren gemäss einem der Ansprüche 1 bis 2, wobei die tertiäre Aminverbindung Triethylamin ist.

4. Verfahren gemäss einem der Ansprüche 1 bis 3, wobei der Palladiumkatalysator ein Palladium-Komplex ist, der aus einem nullwertigen oder zweiwertigen Palladium und einem Liganden auf Basis eines tertiären Phosphins gebildet ist.

5. Verfahren gemäss Anspruch 4, wobei der Palladium-Komplex einer ist, der in einem wasserhaltigen Lösungsmittel durch Zugabe eines Liganden auf Basis eines tertiären Phosphins und einer Verbindung, die als Quelle für ein nullwertiges oder zweiwertiges Palladium dient, zu dem wasserhaltigen Lösungsmittel gebildet wird, oder der Palladium-Komplex einer ist, der zuvor aus einem nullwertigen oder zweiwertigen Palladium und einem Liganden auf Basis eines tertiären Phosphins gebildet worden ist.

6. Verfahren gemäss einem der Ansprüche 1 bis 5, wobei die Reaktion in Gegenwart eines Phasentransferkatalysators durchgeführt wird.

## Revendications

1. Procédé de production d'un composé biaryle contenant du fluor de Formule (III) : 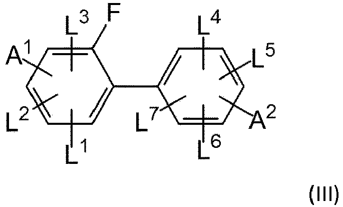 dans lequel
L¹ à L⁷ sont chacun indépendamment H, F ou un alkyle C₁₋₆ linéaire ou ramifié ;
A¹ est H, F, un alkyle C₁₋₆ linéaire ou ramifié, un groupe hydrocarboné aliphatique cyclique ayant facultativement un ou plusieurs substituants, et ayant en outre facultativement un ou plusieurs hétéroatomes, un phényle facultativement substitué, ou un hétérocycle aromatique facultativement substitué, et
A² est H, F, -CN, -CF₃, -OCF₃, -O-A³, -COOA³, -CF₂OA³, ou 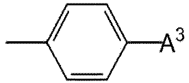 dans lequel A³ est H, un alkyle C₁₋₆ linéaire ou ramifié, un phényle, un difluorophényle, un trifluorophényle, un difluorophényle trifluorométhyle ou un difluorophényle trifluorométhoxy ;
le procédé comprend la réaction d'au moins un composé acide boronique aromatique de formule (I) ou un trimère cyclique (la) formé par la déshydratation-condensation de celui-ci : 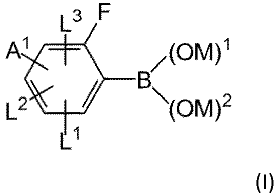 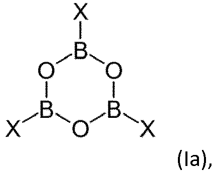 dans lequel X est 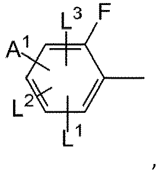 et dans lequel L¹-L³ et A¹ sont les mêmes que définis ci-dessus, et M¹ et M² sont chacun indépendamment H, un alkyle C₁₋₆ linéaire ou ramifié, ou un atome métallique monovalent; ou M¹ et M² se lient l'un à l'autre pour former un groupe hydrocarboné aliphatique bivalent,
avec un chlorure aromatique de formule (II) : 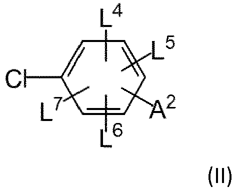 dans lequel L⁴-L⁷ et A² sont les mêmes que définis ci-dessus, dans un solvant contenant de l'eau sélectionné à partir d'eau et de mélanges d'eau et d'un solvant organique, à une température de réaction de 60 °C ou plus et en présence d'un catalyseur au palladium et d'un composé d'amine tertiaire.

2. Procédé selon la revendication 1, dans lequel le composé (I) a la formule suivante, avec L¹-L³ et A¹ étant comme définis dans la revendication 1 : 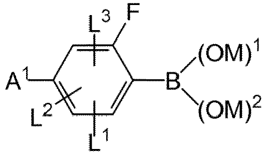 et le composé (II) a la formule suivante, avec L⁴-L⁷ et A² étant comme définis dans la revendication 1 :

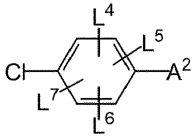

3. Procédé selon l'une quelconque des revendications 1 à 2, dans lequel le composé d'amine tertiaire est une triéthylamine.

4. Procédé selon l'une quelconque des revendications 1 à 3, dans lequel le catalyseur au palladium est un complexe de palladium formé d'un palladium zérovalent ou divalent et d'un ligand à base de phosphine tertiaire.

5. Procédé selon la revendication 4, dans lequel le complexe de palladium est l'un qui est formé dans un solvant contenant de l'eau en ajoutant un ligand à base de phosphine tertiaire et un composé qui sert de source pour un palladium zérovalent ou divalent au solvant contenant de l'eau, ou le complexe de palladium est l'un qui a été formé auparavant d'un palladium zérovalent ou divalent et d'un ligand à base de phosphine tertiaire

6. Procédé selon l'une quelconque des revendications 1 à 5,
dans lequel la réaction est exécutée en présence d'un catalyseur de transfert de phase.
